# EUROPEAN PATENT SPECIFICATION

(11) **EP 4 361 628 B1**
(45) Date of publication and mention of the grant of the patent: **18.02.2026**
(21) Application number: 23198858.5
(22) Date of filing: 21.09.2023
(51) Int. Cl.: G01N 33/00

(54) **METHOD AND SYSTEM FOR FIRMWARE FUNCTIONALITY TESTING OF GAS DETECTOR DEVICES**
VERFAHREN UND SYSTEM ZUR FIRMWARE-FUNKTIONSPRÜFUNG VON GASDETEKTORVORRICHTUNGEN
PROCÉDÉ ET SYSTÈME DE TEST DE FONCTIONNALITÉ DE MICROLOGICIEL DE DISPOSITIFS DÉTECTEURS DE GAZ

(30) Priority: 27.10.2022 IN 202211061202
(43) Date of publication of application: 01.05.2024
(73) Proprietor: Honeywell International Inc., Charlotte, NC 28202 (US)
(72) Inventor: KULKARNI, Sumit Suresh, Charlotte 28202 (US); GEORGE, Renjith, Charlotte 28202 (US); SUBBAIYAN, Jayakumar, Charlotte 28202 (US)
(74) Representative: Haseltine Lake Kempner LLP

(56) References cited:
- DE-A1- 102019 218 325

## Description

### FIELD OF THE INVENTION

The present invention relates to a computer-implemented method for performing firmware functionality testing of a gas detector device, and a gas detector device.

### BACKGROUND

Gas detector devices (including portable gas detectors and transmitters configured to receive input from external gas sensors) are generally tested by product manufacturers during product development and at customer site during proof test (to verify the various functionalities of the gas detector device. However, because testing is generally performed using actual gas, several limitations are posed, including challenges testing critical firmware functionalities. Through applied effort, ingenuity, and innovation, Applicant has solved problems relating to testing gas detector devices by developing solutions embodied in the present disclosure, which are described in detail below.

DE 102019218325 A discloses a test method for a system for detecting a thermal event of an electrical energy storage device in a vehicle.

### BRIEF SUMMARY

The present invention relates to testing gas detector devices. According to an aspect of the invention, there is provided a computer-implemented method for performing firmware functionality testing of a gas detector device, the computer-implemented method comprising for each gas of one or more gases; applying, using one or more processors, selected test data to a firmware of the gas detector device, wherein the selected test data is selected from test data stored locally on the gas detector device, and wherein the selected test data comprises simulated sensor data and is selected based at least in part on the gas; generating, using the one or more processors, one or more output signals based at least in part on processing of the selected test data by the firmware; and generating, using the one or more processors, testing output data based at least in part on comparing the one or more output signals to one or more expected output signals for the selected test data, wherein the testing output data comprise data indicative of performance of one or more firmware functionalities of the firmware with respect to the gas.

In various embodiments, the test data may comprise one or more Analog-to-Digital Converter (ADC) Count values, each corresponding to a gas concentration value.

In various embodiments, processing the selected test data may comprise for each of the one or more ADC count values, generating a gas concentration value for the ADC count values.

In various embodiments, applying the selected test data may comprise: reading the one or more ADC count values for the gas from a test data buffer; and providing the one or more of ADC count values to a gas data processing unit.

In various embodiments, the test data buffer may comprise respective ADC count values for each gas of the one or more gases.

In various embodiments, the method may further comprise generating a test report based at least in part on the testing output data.

In various embodiments, the one or more output signals may correspond to one or more of: (i) alarm level, (ii) vibration level, or (iii) light indicator pattern.

In various embodiments, the method may further comprise applying the selected test data in response to receiving a test mode signal.

According to another aspect of the invention there is provided a gas detector device comprising: a test driver unit, wherein the test driver unit is configured to: for each gas of one or more gases; apply selected test data to a firmware of the gas detector device, wherein the selected test data comprise simulated sensor data and is selected based at least in part on the gas; generate one or more output signals based at least in part on processing the selected test data by the firmware; and generate testing output data based at least in part on comparing the one or more output signals to one or more expected output signals for the selected test data, wherein the testing output data comprise data indicative of performance of one or more firmware functionalities of the firmware with respect to the gas.

In various embodiments, the selected test data comprise one or more Analog-to-Digital Converter (ADC) Count values, each corresponding to a gas concentration value.

In various embodiments, processing the selected test data comprise: for each of the one or more ADC count values, generating a gas concentration value based at least in part on the ADC count values.

In various embodiments, applying the selected test data for a particular gas may comprise reading the one or more ADC count values for the gas from a test data buffer; and providing the one or more ADC count values to a gas data processing unit.

In various embodiments, the test data buffer may comprise the ADC count values for each gas of the one or more gases.

In various embodiments, the test driver unit may be configured to generate a test report based at least in part on the testing output data.

In various embodiments, the one or more output signals may correspond to one or more of: (i) alarm level, (ii) vibration level, or (iii) light indicator pattern.

In various embodiments, the test driver unit is configured to apply the test data in response to receiving a test mode signal.

### BRIEF DESCRIPTION OF THE DRAWINGS

Reference will now be made to the accompanying drawings, which are not necessarily drawn to scale, and wherein:
Figure 1A illustrates an operational example of a gas detector device and associated gas sensor in accordance with one or more embodiments of the present disclosure.
Figure 1B schematically illustrates an exemplary block diagram of a control system of a gas detector device in accordance with one or more embodiments of the present disclosure.
Figure 2 schematically illustrates an exemplary process flow of operations performed by a gas detector device in test mode in accordance with one or more embodiments of the present disclosure.
Figure 3 schematically illustrates an exemplary process flow for performing firmware functionality testing of an exemplary gas detector device in accordance with various embodiments of the present invention.

### DETAILED DESCRIPTION

The present disclosure more fully describes various embodiments with reference to the accompanying drawings. It should be understood that some, but not all embodiments are shown and described herein. Indeed, the embodiments may take many different forms, and accordingly this disclosure should not be construed as limited to the embodiments set forth herein. Rather, these embodiments are provided so that this disclosure will satisfy applicable legal requirements. Like numbers refer to like elements throughout.

It should be understood at the outset that although illustrative implementations of one or more aspects are illustrated below, the disclosed systems, and methods may be implemented using any number of techniques, whether currently known or not yet in existence. The disclosure should in no way be limited to the illustrative implementations, drawings, and techniques illustrated below, but may be modified within the scope of the appended claims. While values for dimensions of various elements are disclosed, the drawings may not be to scale.

The words "example," or "exemplary," when used herein, are intended to mean "serving as an example, instance, or illustration." Any implementation described herein as an "example" or "exemplary embodiment" is not necessarily preferred or advantageous over other implementations.

Described herein are methods, systems, and apparatuses for testing firmware functionalities of gas detector devices. Examples of firmware functionality tests that may be performed according to one or more embodiments of the present disclosure include, but not limited to, threshold alarm test to determine whether an alarm of a gas detector is triggered when the gas concentration exceeds a defined threshold, short-term exposure limit (STEL) alarm test to determine whether an alarm of a gas detector device is triggered when the average gas concentration over a defined time period (generally short time period) exceeds a defined threshold, long-term exposure limit (STEL) to determine whether an alarm of a gas detector device is triggered when the average gas concentration over a defined time period (generally long time period) exceeds a defined threshold, gas rate test, gas over range test, threshold alarm test, time weighted alarm test, rate alarm test, gas and signal over and under range test, buzzer test, light emitting diode (LED) test, vibration test, buzzer functionality test to determine whether a given buzzer of a gas detector device is triggered as expected, vibration response, event history, and/or the like.

Many gas detector devices are configured to support multiple gas sensors which detect hundreds of gases, and it is often desired to test the various firmware functionalities of a gas detector device with respect to each of the different gases (e.g., carbon monoxide, hydrogen sulfide, ammonia, combustible gases (LEL), nitrogen dioxide, and/or the like) supported, at least during product development. However, testing the various functionalities of gas detector devices generally require using actual gas, and because of several reasons, including, but not limited to, high toxicity of these gases, environmental pollution concerns, gas cost, infrastructure limitations (e.g., scrubber compatibility), and difficulty simulating certain conditions (e.g., sensor degradation), it is often challenging to test several critical firmware functionalities of gas detector devices.

To overcome the above problems, various embodiments, of the present disclosure provide methods, systems, and apparatuses for testing firmware functionalities of gas detector devices that eliminates the need to use actual gas by storing test data (Analog-to-Digital Converter (ADC) count values) representative of gas concentration values for each gas range locally on the gas detector device, and simulating gas sensor data during testing using a subset (e.g., one, some, or all) of the test data (e.g., selected test data). Many gas sensors generate analog signals (corresponding to gas concentrations) that are received by a gas detector device. These analog signals are generally converted into digital signals (e.g., using Analog-to-Digital Converters) represented as Analog-to-Digital (ADC) Count Values and processed to determine the gas concentration of a given gas based at least in part on the ADC count value. By using simulated gas sensor data (e.g., pre-determined ADC count values for different gases) during testing to test firmware functionality of gas detector devices (as opposed to receiving analog sensor signals generated based at least in part on actual gases), various embodiments according to the present disclosure enable testing of firmware functionalities of gas detector devices without using actual gas. Accordingly, various techniques discussed herein (utilizing simulated gas sensor data) improve firmware functionality testing of gas detector devices, including, but not limited, to reduced cost, increased testing capacity, and reduced hazardous risks. Moreover, using simulated gas sensor data enables testing of certain functionalities (e.g., sensor degradation) that would otherwise be challenging or impossible to test.

An exemplary gas detector device according to one or more embodiments described herein includes: (i) a test data buffer configured to store test data (ADC count values corresponding to gas concentration values) for testing firmware functionalities of the exemplary gas detector device, and (ii) a test driver unit configured to control the operation of the exemplary gas detector device when the gas detector device is in test mode.

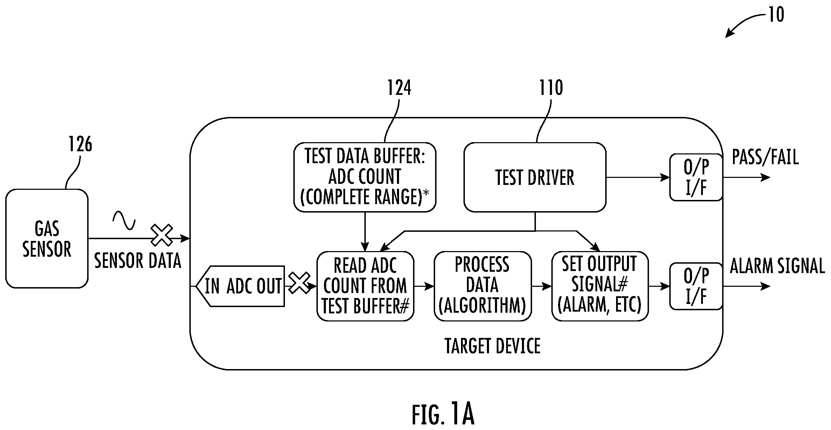

FIG. 1A depicts an operational example of a an exemplary gas detector device 10 in test mode. As shown in FIG. 1A, the gas detector device includes a test driver unit 110 and a test data buffer 124 comprising test data (ADC count values) that encompass the full range of gas concentration (e.g., ppm) for each gas supported by the exemplary gas detector device 10.

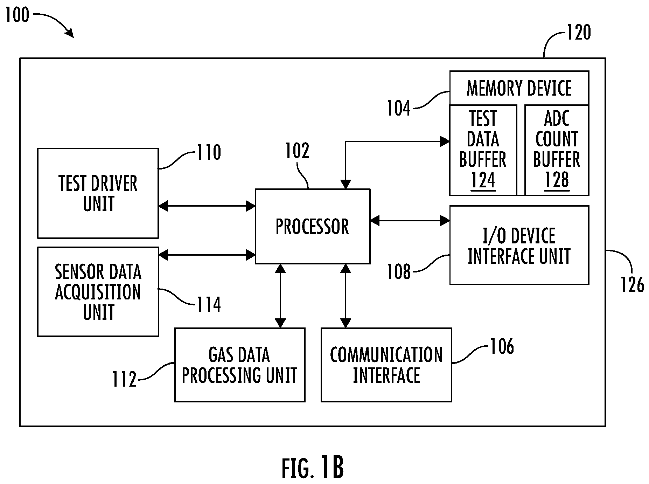

In an example embodiment, the test data buffer 124 of an exemplary gas detector device 10 includes one or more ADC count values (e.g., a series of ADC count values, a set of ADC count values, or the like) for each gas, and for each gas, the one or more ADC count values for the gas may encompass a full range of gas concentration for the gas. As further shown in FIG. 1B, during testing, data acquisition from a gas sensor 126 associated with the exemplary gas detector device 10 may be disabled. Additionally and/or alternatively, the Analog-to-Digital Converter for generating ADC count values (e.g., based at least in part on input data received from the gas sensor 126) during normal operation may be disabled.

In one or more embodiments, the test driver unit 110 is configured to simulate gas sensor data based at least in part on the functionality being tested and/or gas being tested, and apply the simulated gas sensor data (e.g., selected test data), wherein the simulated gas sensor data (e.g., selected test data), is processed by the gas detector device firmware to generate one or more output signals. Selected test data may describe a subset of the test data (e.g., a subset of the ADC count values that may be stored locally on the gas detector device), wherein each selected test data may be selected based at least in part on the gas and/or the functionality being tested. For example, given a first gas and a second gas, each supported by an exemplary gas detector device, a selected test data for the first gas may comprise ADC count values of [34760, 33764, 32768, 12845] corresponding to gas concentration of [-10, -5, 0, 100 ], and a selected test data for the second gas may comprise ADC count values of [34297, 32768, 1998, 14424] corresponding to gas concentration of [-2.5, 0, 20.9, 25, 30] respectively. It should be understood that this disclosure should in no way be limited to the above are examples. In one or more embodiments, selected test data (e.g., simulated sensor data) comprising ADC count values may be applied in a stepwise fashion (e.g., from low gas concentration to high gas concentration). Additionally and/or alternatively, in one or more embodiments, selected test data (comprising ADC count values) may be applied in one or more loops/cycles of low gas concentration to high gas concentration and continued from high gas concentration to low gas concentration. In one or more embodiments, a given selected test data may describe a firmware functionality test scenario, and an exemplary gas detector device 10 may store (e.g., test driver unit thereof) a plurality of selected test data, each corresponding to a firmware functionality test scenario of a plurality of firmware functionality test scenarios.

In one or more embodiments, the manner in which test data is applied may be configurable. Additionally and/or alternatively, in one or more embodiments, the selected test data may be configurable. For example, the selected test data for a given gas and/or functionality being tested may comprise a configurable number/units of ADC count values (e.g., 10 ADC count values, 15 ADC count values, and/or the like). In one or more embodiments, for each selected test data, the test driver unit 110 stores expected output of the gas detector device firmware in response the selected test data. An expected output for a given selected test data may describe output data and/or corresponding event (e.g., alarm of a given level, buzzer of a given level, light of a given intensity, flashing light of a given pattern, data verification, data sequence and data priority send to external devices after particular events, state of relay & digital output, analog output and/or the like) a gas detector device is configured to trigger in response to a given ADC count value and/or sequence of ADC count values with respect to one or more defined thresholds. In one or more embodiments, the test driver unit 110 applies the selected test data, where the selected test data is processed to generate one or more output signals. In one or more embodiments, the test driver unit 110 may: (i) monitor the applied selected test data (e.g., step-wise application of the ADC count values), (ii) measure the output of the firmware in response to the applied selected test data, (iii) and compare the output of the firmware with the expected output, where matching output may be indicative that the firmware passed the corresponding functionality test, and a non-matching output may be indicative that the firmware failed the functionality test. In some embodiments, as shown in FIG. 1A, an exemplary gas detector device 10 (e.g., test driver unit 110 thereof) may be configured to generate a PASS/FAIL output for each functionality tested. In one or more embodiments, the PASS/FAIL output for each functionality test performed may be generated as a report. Additionally, in some embodiments, the generated report and/or PASS/FAIL output may be transmitted to one or more computing devices. Additionally and/or alternatively, the result from comparing the output signal of the firmware in response to applied selected test data (e.g., simulated sensor data) may be stored locally on the firmware, where the stored result may be accessible. In one or more embodiments, expected output of a firmware in response to the applied test data may include one or more of alarm level, vibration level, or light (e.g., LED) level.

FIG. 1B depicts a block diagram of an exemplary control system 100 of an exemplary gas detector device 10 according to one or more embodiments described herein. In an example embodiment, the control system 100 includes a processor 102, a memory device 104, a communication interface 106, an input/output (I/O) device interface unit 108, a test driver unit 110, and a gas data processing unit 112. In an example embodiment, the processor 102 may be communicatively coupled to each of the memory device 104, the communication interface 106, the I/O device interface unit 108, the test driver unit 110, gas data processing unit 112, and sensor data acquisition unit 114.

The processor 102 may be embodied as a means including one or more microprocessors with accompanying digital signal processor(s), one or more processor(s) without an accompanying digital signal processor, one or more coprocessors, one or more multicore processors, one or more controllers, processing circuitry, one or more computers, various other processing elements including integrated circuits such as, for example, an application specific integrated circuit (ASIC) or field programmable gate array (FPGA), or some combination thereof. Accordingly, although illustrated in FIG. 1B as a single processor, in an example embodiment, the processor 102 may include a plurality of processors and signal processing modules. The plurality of processors may be embodied on a single electronic device or may be distributed across a plurality of electronic devices collectively configured to function as the circuitry of the control system 100. The plurality of processors may be in operative communication with each other and may be collectively configured to perform one or more functionalities of the circuitry of the control system 100, as described herein. In an example embodiment, the processor 102 may be configured to execute instructions stored in the memory device 104 or otherwise accessible to the processor 102. These instructions, when executed by the processor 102, may cause the circuitry of the control system 100 to perform one or more of the functionalities, as described herein.

Whether configured by hardware, firmware/software methods, or by a combination thereof, the processor 102 may include an entity capable of performing operations according to embodiments of the present disclosure while configured accordingly. Thus, for example, when the processor 102 is embodied as an ASIC, FPGA or the like, the processor 102 may include specifically configured hardware for conducting one or more operations described herein. Alternatively, as another example, when the processor 102 is embodied as an executor of instructions, such as may be stored in the memory device 104, the instructions may specifically configure the processor 102 to perform one or more algorithms and operations described herein.

Thus, the processor 102 used herein may refer to a programmable microprocessor, microcomputer or multiple processor chip or chips that can be configured by software instructions (applications) to perform a variety of functions, including the functions of the various embodiments described above. In some devices, multiple processors may be provided dedicated to wireless communication functions and one processor dedicated to running other applications. Software applications may be stored in the internal memory before they are accessed and loaded into the processors. The processors may include internal memory sufficient to store the application software instructions. In many devices, the internal memory may be a volatile or nonvolatile memory, such as flash memory, or a mixture of both. The memory can also be located internal to another computing resource (e.g., enabling computer readable instructions to be downloaded over the Internet or another wired or wireless connection).

The memory device 104 may include suitable logic, circuitry, and/or interfaces that are adapted to store a set of instructions that is executable by the processor 102 to perform predetermined operations. Some of the commonly known memory implementations include, but are not limited to, a hard disk, random access memory, cache memory, read only memory (ROM), erasable programmable read-only memory (EPROM) & electrically erasable programmable read-only memory (EEPROM), flash memory, magnetic cassettes, magnetic tape, magnetic disk storage or other magnetic storage devices, a compact disc read only memory (CD-ROM), digital versatile disc read only memory (DVD-ROM), an optical disc, circuitry configured to store information, or some combination thereof. In an example embodiment, the memory device 104 may be integrated with the processor 102 on a single chip, without departing from the scope of the disclosure. In one or more embodiments, the memory device 104 includes a test data buffer 124 configured to store test data (e.g. ADC count values) for testing various firmware functionalities of the gas detector device 10. In one or more embodiments, the test data buffer 124 stores test data that includes for each gas type supported by the gas detector device10, Analog-to-Digital Converter (ADC) count values representative of a full range (e.g., 0%- 100%) of the gas.

The communication interface 106 may correspond to a communication interface that may facilitate transmission and reception of messages and data to and from various devices. For example, the communication interface 106 may be communicatively coupled to one or more computing devices 120. Examples of the communication interface 106 may include, but are not limited to, an antenna, an Ethernet port, a USB port, a serial port, or any other port that can be adapted to receive and transmit data (e.g., via at least one wired and/or wireless protocol). The communication interface 106 transmits and receives data and/or messages in accordance with the various communication protocols, such as, I2C, TCP/IP, UDP, and 4G, 4G, or 4G communication protocols.

The I/O device interface unit 108 may include suitable logic and/or circuitry that may be configured to communicate with the one or more components of the gas detector device 10, in accordance with one or more device communication protocols such as, but not limited to, I2C communication protocol, Serial Peripheral Interface (SPI) communication protocol, Serial communication protocol, Control Area Network (CAN) communication protocol, and 1-Wire^{®} communication protocol. In an example embodiment, the I/O device interface unit 108 may communicate with one or more gas sensors 126. For example, the I/O device interface unit 108 may receive input data from the gas sensor 126. Some examples of the I/O device interface unit 108 may include, but not limited to, a Data Acquisition (DAQ) card, an electrical drives driver circuit, and/or the like.

The sensor data acquisition unit 114 may include suitable logic and/or circuitry for receiving gas sensor data from one or more gas sensors 126 during normal operation. During testing, the sensor data acquisition unit 114 may be disabled. The sensor data acquisition unit 114 may include an ADC converter for converting analog output signals from the one or more gas sensors 126 to digital signals (e.g., ADC count values) during normal operation. During testing, the ADC converter may be disabled.

The gas data processing unit 112 may include suitable logic and/or circuitry that may cause the gas detector device 10 to perform gas data processing operation to generate one or more output signals which may be indicative of the presence or absence of gas during normal operation, and/or indicative of firmware functionality. In an example embodiment, during normal operation, the gas data processing unit 112 may perform gas data processing operation based at least on input data (e.g., analog signal) received from the one more gas sensors 126, and during testing, may perform gas data processing operation based at least in part on test data from the test data buffer 124. In one or more embodiments, during gas data processing operation, the gas data processing unit 112 may be configured to receive ADC count values originating from the ADC count buffer 128. In one or more embodiments, the gas data processing by the gas data processing unit 112 may include converting the ADC count values into corresponding gas concentration value, either originating from the test data buffer or the ADC count buffer.

The test driver unit 110 may include suitable logic and/or circuitry for testing the gas detector device 10, and may cause the gas detector device 10 to operate in a test mode (e.g., based at least in part on one or more input signals (e.g., test mode input signals). In an example embodiment, the test driver unit 110 may be configured to store various parameters for testing the functionalities of the gas detector device 10. In an example embodiment, the test driver unit 110 may be configured to store (e.g., in the memory device 104) parameters that may include, but not limited to data representative of various functionality tests (e.g., various firmware functionality test scenarios) that may be performed by the corresponding gas detector device 10, and criteria for each functionality test. In one or more embodiments, the criteria may include for each gas type, the ADC count values (e.g., selected test data) for testing a particular gas and/or particular firmware functionality, and may include the sampling rate for the ADC count values. Additionally, in one or more embodiments, the test driver unit 110 may be configured to store (e.g., in the memory device 104), the corresponding gas concentration values represented by each ADC count value for each gas, the expected output signals by the gas detector device firmware in response to a given applied selected test data (e.g., ADC count values), and expected triggered events by the gas detector device firmware in response to a given selected test data. (e.g., corresponding to one or more functionality tests). Further, the test driver unit 110 may be configured to control the ADC count values processed by the gas data processing unit 112 when the gas detector device 10 is in a test mode.

In an example embodiment, the test driver unit 110 may be configured to generate testing output data based at least in part on output of the gas detector device firmware (e.g., received from the memory device 104) in response to a given selected data (e.g., ADC count values). In some embodiments, the test driver unit 110 may measure the output of the firmware of the gas detector device. In an example embodiment, the testing output data may include data representative of the performance of the gas detector device 10 with respect to one or more firmware functionalities of the gas detector device based at least in part on comparing the measured output data or output data retrieved from the memory device 104 (corresponding to the response of the gas detector device 10 to the test data) with the expected output values. In some embodiments, the testing output data may include data indicating whether the firmware of the gas detector device 10 passed or failed a given firmware functionality test. In some embodiments, the test driver unit 110 may be configured to transmit the testing output data to one or more computing devices 120. Additionally and/or alternatively, in an example, embodiment, the test driver unit 110 may be configured to store the testing output data in the memory device 104.

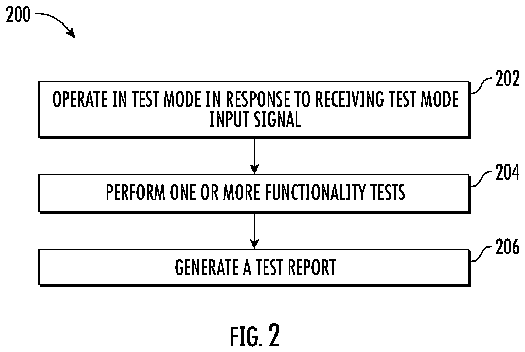

FIG. 2, illustrates an example flowchart of the operations performed by an apparatus such as the exemplary gas detector device 10 in a test mode. It will be understood that each block of the flowcharts, and combinations of blocks in the flowcharts, may be implemented by various means, such as hardware, firmware, one or more processors, circuitry and/or other devices associated with execution of software including one or more computer program instructions. For example, one or more of the procedures described above may be embodied by computer program instructions. In this regard, the computer program instructions which embody the procedures described above may be stored by a memory of an apparatus employing an embodiment of the present invention and executed by a processor in the apparatus. As will be appreciated, any such computer program instructions may be loaded onto a computer or other programmable apparatus (e.g., hardware) to produce a machine, such that the resulting computer or other programmable apparatus provides for implementation of the functions specified in the flowcharts' block(s). These computer program instructions may also be stored in a non-transitory computer-readable storage memory that may direct a computer or other programmable apparatus to function in a particular manner, such that the instructions stored in the computer-readable storage memory produce an article of manufacture, the execution of which implements the function specified in the flowcharts' block(s). The computer program instructions may also be loaded onto a computer or other programmable apparatus to cause a series of operations to be performed on the computer or other programmable apparatus to produce a computer-implemented process such that the instructions which execute on the computer or other programmable apparatus provide operations for implementing the functions specified in the flowcharts' block(s). As such, the operations of FIG. 2, when executed, convert a computer or processing circuitry into a particular machine configured to perform an example embodiment of the present invention. Accordingly, the operations of FIG. 2, define an algorithm for configuring a computer or processor, to perform an example embodiment. In some cases, a general-purpose computer may be provided with an instance of the processor which performs the algorithm of FIG. 2 to transform the general-purpose computer into a particular machine configured to perform an example embodiment.

Accordingly, blocks of the flowchart support combinations of means for performing the specified functions and combinations of operations for performing the specified functions. It will also be understood that one or more blocks of the flowcharts', and combinations of blocks in the flowchart, can be implemented by special purpose hardware-based computer systems which perform the specified functions, or combinations of special purpose hardware and computer instructions.

The foregoing method descriptions and operations described in the flowchart 200 illustrated in FIG. 2 is provided merely as illustrative example and is not intended to require or imply that the steps of the various embodiments must be performed in the order presented. As will be appreciated by one of skill in the art, the order of steps in these embodiments may be performed in different orders.

At step/operation 202, in response to receiving a test mode input signal indicating selection of a test mode, the gas detector device 10 may be operated in a test mode to test one or more functionalities of the firmware of the gas detector device 10. The testing may include for a given firmware functionality of the gas detector device 10, determining whether the output of the firmware in response to applied selected test data (e.g., ADC count values) matches the expected output. An exemplary gas detector device 10 may have one or more test modes (e.g., firmware test mode, proof test firm mode) and/or sub-modes. In one or more embodiments, the gas detector device 10 may be configured to perform one or more firmware functionality tests based at least in part on a selected test mode.

In one or more embodiments, in response to receiving the test mode input signal indicating selection of a test mode, the gas detector device 10 using, the test driver unit 110, may cause the gas detector device 10 to disable receiving input data from the one or more gas sensors associated with the gas detector device 10. Additionally, in some embodiments, the test driver unit 110 may control the operation of the gas detector device firmware while in a testing mode.

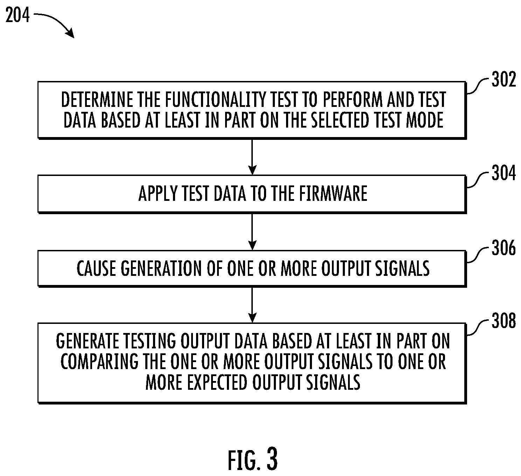

At step/operation 204, the gas detector device 10 using the test driver unit 110, performs one or more functionality tests. In one or more embodiments, the step/operation 204 may be performed in accordance with the operation that is depicted in FIG. 3. The process that is depicted in FIG. 3 begins at step/operation 302 when the gas detector device 10 using the test driver unit 110 determines, based at least in part on the selected test mode, one or more functionality tests to perform and data (e.g., selected test data) to utilize in performing the one or more functionality tests. For example, a user may select a test mode where testing of each gas supported by the gas detector device 10 is performed. As another example, a user may select a test mode where testing of a subset of the gases supported by the gas detector device 10 is performed. As yet another example, a user may select a test mode where testing of each functionality of the firmware is performed. As a further example, a user may select a test mode where testing of a subset of the functionalities of the firmware.

For each gas being tested, the gas detector device 10 using the test driver unit 110, based at least in part on the selected test mode, determines the selected test data (e.g., corresponding ADC count values) for the one or more functionality tests for the given gas. In an example embodiment, the selected test data includes the ADC count values corresponding to the simulated sensor data for the given gas. Additionally, in some embodiments, the gas detector device 10, using the test driver unit 110 may determine the sampling rate for the ADC count values. In one or more embodiments, the sampling rate for a given gas during test mode of the gas detector device may be the same as the sampling rate during normal operation mode.

At step/operation 304, the gas detector device 10 using the test driver unit 110, applies the test data (e.g., ADC count values) to the firmware. In one or more embodiments, applying the test data may comprise retrieving (e.g., reading) the ADC count values for the given gas from a test data buffer 124 and providing (e.g., feeding) the ADC count values to a gas data processing unit 112 of the gas detector device 10 (e.g., in a stepwise fashion). The test data buffer 124 may be configured to store ADC count values that encompasses complete gas range for each gas supported by the gas detector device 10.

At step/operation 306, the gas detector device 10, generates one or more output signals based at least in part on the ADC count values. For example, in one or more embodiments, the gas detector device 10 (e.g., using the test driver unit 110) causes the gas data processing unit 112 to process the selected test data (e.g., selected ADC count values) to generate the one or more output signals based at least in part on the selected test data. In one or more embodiments, processing the test data may comprise for each of the one or more ADC count values of the selected test data, generating a gas concentration value based at least in part on the ADC count values. In some embodiments, the one or more output signals corresponds to one or more of: (i) alarm level, (ii) vibration level, (iii) light indicator pattern, (iv) relay output level, (v) digital output level, (vi) analog output level, (vii) data, or (viii) data-sequence and data priority sent to external device.

At step/operation 308, the gas detector device 10 using the test driver unit 110 generates testing output data based at least in part on comparing the one or more output signals to one or more expected output signals for the test data. For example, for a given selected test data, the gas detector device may be configured to generate output data and/or trigger one or more events (e.g., alarm, buzzer, light, and/or the like) in response to the applied test data (e.g., when a given ADC count value of the applied ADC count values exceeds a defined threshold). In various embodiments, the testing output data comprise data indicative of the performance of the one or more functionalities of the gas detector device firmware with respect to a given gas. In an example embodiment, the gas detector device 10 using the test driver unit 110 monitors the output of the firmware. For example, for a given selected test data (e.g., selected ADC count values) processed by the gas data processing unit 112, the test driver unit 110 monitors the applied ADC count values and measures the outputs (e.g., LED pattern, Audio level, Vibration pattern, and/or the like), and compares the measured values to the expected output.

Returning to step/operation 206, the gas detector device 10, using the test driver unit 110 generates a test report based at least in part on the testing output data. For example, the test report may comprise the testing output data indicating whether the firmware failed or passed a given functionality test. In one or more embodiments, the gas detector device 10, using the test driver unit 110 may transmit the testing output data to one or more computing devices.

Many modifications and other embodiments will come to mind to one skilled in the art to which this disclosure pertains having the benefit of the teachings presented in the foregoing descriptions and the associated drawings. Although specific terms are employed herein, they are used in a generic and descriptive sense only and not for purposes of limitation.

## Claims

1. A computer-implemented method for performing firmware functionality testing of a gas detector device (10), the computer-implemented method comprising:
for each gas of one or more gases;
applying (302), using one or more processors (102), selected test data to a firmware of the gas detector device (10), wherein the selected test data is selected from test data stored locally on the gas detector device (10), and wherein the selected test data comprises simulated sensor data and is selected based at least in part on the gas;
generating (306), using the one or more processors (102), one or more output signals based at least in part on processing of the selected test data by the firmware; and
generating (308), using the one or more processors (102), testing output data based at least in part on comparing the one or more output signals to one or more expected output signals for the selected test data, wherein the testing output data comprise data indicative of performance of one or more firmware functionalities of the firmware with respect to the gas.

2. The computer-implemented method of claim 1, wherein the test data comprises one or more Analog-to-Digital Converter, ADC, count values, each corresponding to a gas concentration value.

3. The computer-implemented method of claim 1, wherein processing of the selected test data comprises:
for each of the one or more ADC count values, generating a gas concentration value for the ADC count values.

4. The computer-implemented method of claim 2, wherein applying the selected test data comprises:
reading the one or more ADC count values for the gas from a test data buffer (124); and
providing the one or more ADC count values to a gas data processing unit (112) comprising the firmware.

5. The computer-implemented method of claim 4, wherein the test data buffer (124) comprises respective ADC count values for each gas of the one or more gases.

6. The computer-implemented method of claim 1, further comprising generating a test report based at least in part on the testing output data.

7. The computer-implemented method of claim 1, wherein the one or more output signals corresponds to one or more of: alarm level, vibration level, or light indicator pattern.

8. The computer-implemented method of claim 1, wherein applying the selected test data comprises applying the selected test data in response to receiving a test mode signal.

9. A gas detector device (10) comprising:
a test driver unit (110), wherein the test driver unit (110) is configured to:
for each gas of one or more gases;
apply selected test data to a firmware of the gas detector device (10), wherein the selected test data comprise simulated sensor data and is selected based at least in part on the gas;
generate one or more output signals based at least in part on processing the selected test data by the firmware; and
generate testing output data based at least in part on comparing the one or more output signals to one or more expected output signals for the selected test data, wherein the testing output data comprise data indicative of performance of one or more firmware functionalities of the firmware with respect to the gas.

10. The gas detector device (10) of claim 9, wherein the selected test data comprise one or more Analog-to-Digital Converter, ADC, count values, each corresponding to a gas concentration value.

11. The gas detector device (10) of claim 9, wherein processing the selected test data comprise:
for each of the one or more ADC count values, generating a gas concentration value based at least in part on the ADC count values.

12. The gas detector device (10) of claim 10, wherein applying the selected test data for a particular gas comprise:
reading the one or more ADC count values for the gas from a test data buffer (124) comprising the ADC count values for each gas of the one or more gases; and
providing the one or more ADC count values to a gas data processing unit (112) comprising the firmware.

13. The gas detector device (10) of claim 9, wherein the test driver unit (110) is configured to generate a test report based at least in part on the testing output data.

14. The gas detector device (10) of claim 9, wherein the one or more output signals correspond to one or more of: alarm level, vibration level, or light indicator pattern.

15. The gas detector device (10) of claim 9, wherein the test driver unit (110) is configured to apply the selected test data in response to receiving a test mode signal.

## Patentansprüche

1. Computerimplementiertes Verfahren zum Durchführen von Firmware-Funktionsprüfungen einer Gasdetektorvorrichtung (10), das computerimplementierte Verfahren umfassend:
für jedes Gas von einem oder mehreren Gasen:
Anwenden (302), unter Verwendung von einem oder mehreren Prozessoren (102), von ausgewählten Prüfdaten auf eine Firmware der Gasdetektorvorrichtung (10), wobei die ausgewählten Prüfdaten ausgewählt sind aus Prüfdaten, die lokal in der Gasdetektorvorrichtung (10) gespeichert sind, und wobei die ausgewählten Prüfdaten simulierte Sensordaten umfassen und mindestens teilweise auf Basis des Gases ausgewählt sind;
Erzeugen (306), unter Verwendung des einen oder der mehreren Prozessoren (102), eines oder mehrerer Ausgabesignale auf Basis mindestens teilweise des Verarbeitens der ausgewählten Prüfdaten durch die Firmware; und
Erzeugen (308), unter Verwendung des einen oder der mehreren Prozessoren (102), von Prüfungsausgabedaten auf Basis mindestens teilweise des Vergleichens des einen oder der mehreren Ausgabesignale mit einem oder mehreren erwarteten Ausgabesignalen für die ausgewählten Prüfdaten, wobei die Prüfungsausgabedaten Daten umfassen, die anzeigend für eine Leistung von einer oder mehreren Firmwarefunktionen der Firmware in Bezug auf das Gas sind.

2. Computerimplementiertes Verfahren nach Anspruch 1, wobei die Prüfdaten einen oder mehrere Analog-Digital-Wandler-, ADC-, Zählwerte umfassen, von denen jeder einem Gaskonzentrationswert entspricht.

3. Computerimplementiertes Verfahren nach Anspruch 1, wobei das Verarbeiten der ausgewählten Prüfdaten Folgendes umfasst:
für jeden von dem einen oder den mehreren ADC-Zählwerten, Erzeugen eines Gaskonzentrationswertes für die ADC-Zählwerte.

4. Computerimplementiertes Verfahren nach Anspruch 2, wobei das Anwenden der ausgewählten Prüfdaten Folgendes umfasst:
Auslesen des einen oder der mehreren ADC-Zählwerte für das Gas aus einem Prüfdatenpuffer (124); und
Bereitstellen des einen oder der mehreren ADC-Zählwerte an eine Gasdatenverarbeitungseinheit (112), die die Firmware umfasst.

5. Computerimplementiertes Verfahren nach Anspruch 4, wobei der Prüfdatenpuffer (124) jeweilige ADC-Zählwerte für jedes Gas von dem einen oder den mehreren Gasen umfasst.

6. Computerimplementiertes Verfahren nach Anspruch 1, ferner umfassend Erzeugen eines Prüfberichts auf Basis mindestens teilweise der Prüfungsausgabedaten.

7. Computer-implementiertes Verfahren nach Anspruch 1, wobei das eine oder die mehreren Ausgabesignale einem oder mehreren aus Folgendem entsprechen: Alarmstufe, Vibrationsstufe oder Lichtindikatormuster.

8. Computerimplementiertes Verfahren nach Anspruch 1, wobei das Anwenden der ausgewählten Prüfdaten das Anwenden der ausgewählten Prüfdaten als Antwort auf das Empfangen eine Prüfmodussignals umfasst.

9. Gasdetektorvorrichtung (10), umfassend:
eine Prüftreibereinheit (110), wobei die Prüftreibereinheit (110) dazu konfiguriert ist:
für jedes Gas von dem einen oder den mehreren Gasen;
ausgewählte Prüfdaten auf eine Firmware der Gasdetektorvorrichtung (10) anzuwenden, wobei die ausgewählten Prüfdaten simulierte Sensordaten umfassen und mindestens teilweise auf Basis des Gases ausgewählt sind;
ein oder mehrere Ausgabesignale auf Basis mindestens teilweise des Verarbeitens der ausgewählten Prüfdaten durch die Firmware zu erzeugen; und
Prüfungsausgabedaten auf Basis mindestens teilweise des Vergleichens des einen oder der mehreren Ausgabesignale mit einem oder mehreren erwarteten Ausgabesignalen für die ausgewählten Prüfdaten zu erzeugen, wobei die Prüfungsausgabedaten Daten umfassen, die anzeigend für eine Leistung von einer oder mehreren Firmwarefunktionen der Firmware in Bezug auf das Gas sind.

10. Gasdetektorvorrichtung (10) nach Anspruch 9, wobei die ausgewählten Prüfdaten einen oder mehrere Analog-Digital-Wandler-, ADC-, Zählwerte umfassen, von denen jeder einem Gaskonzentrationswert entspricht.

11. Gasdetektorvorrichtung (10) nach Anspruch 9, wobei das Verarbeiten der ausgewählten Prüfdaten Folgendes umfasst:
für jeden von dem einen oder den mehreren ADC-Zählwerten, Erzeugen eines Gaskonzentrationswertes auf Basis mindestens teilweise der ADC-Zählwerte.

12. Gasdetektorvorrichtung (10) nach Anspruch 10, wobei das Anwenden der ausgewählten Prüfdaten für ein bestimmtes Gas Folgendes umfasst:
Auslesen des einen oder der mehreren ADC-Zählwerte für das Gas aus einem Prüfdatenpuffer (124), die die ADC-Zählwerte für jedes Gas von dem einen oder den mehreren Gasen umfassen; und
Bereitstellen des einen oder der mehreren ADC-Zählwerte an eine Gasdatenverarbeitungseinheit (112), die die Firmware umfasst.

13. Gasdetektorvorrichtung (10) nach Anspruch 9, wobei die Prüftreibereinheit (110) dazu konfiguriert ist, einen Prüfbericht auf Basis mindestens teilweise der Prüfungsausgabedaten zu erzeugen.

14. Gasdetektorvorrichtung (10) nach Anspruch 9, wobei das eine oder die mehreren Ausgabesignale einem oder mehreren aus Folgendem entsprechen: Alarmstufe, Vibrationsstufe oder Lichtindikatormuster.

15. Gasdetektorvorrichtung (10) nach Anspruch 9, wobei die Prüftreibereinheit (110) dazu konfiguriert ist, die ausgewählten Prüfdaten als Antwort auf das Empfangen eine Prüfmodussignals anzuwenden.

## Revendications

1. Procédé mis en œuvre par ordinateur pour l'exécution de tests de fonctionnalité du micrologiciel d'un dispositif détecteur de gaz (10), le procédé mis en œuvre par ordinateur comprenant :
pour chaque gaz parmi un ou plusieurs gaz ;
l'application (302), à l'aide d'un ou plusieurs processeurs (102), de données de test sélectionnées à un micrologiciel du dispositif détecteur de gaz (10), dans lequel les données de test sélectionnées sont sélectionnées parmi des données de test stockées localement sur le dispositif détecteur de gaz (10), et dans lequel les données de test sélectionnées comprennent des données de capteur simulées et sont sélectionnées au moins en partie en fonction du gaz ;
la génération (306), à l'aide du ou des processeurs (102), d'un ou plusieurs signaux de sortie sur la base, au moins en partie, du traitement des données de test sélectionnées par le micrologiciel ; et
la génération (308), à l'aide du ou des processeurs (102), de données de test de sortie sur la base, au moins en partie, d'une comparaison du ou des signaux de sortie avec un ou plusieurs signaux de sortie attendus pour les données de test sélectionnées, dans lequel les données de test de sortie comprennent des données indicatives des performances d'une ou plusieurs fonctionnalités du micrologiciel par rapport au gaz.

2. Procédé mis en œuvre par ordinateur selon la revendication 1, dans lequel les données de test comprennent une ou plusieurs valeurs de comptage de convertisseur analogique-numérique, ADC, chacune correspondant à une valeur de concentration de gaz.

3. Procédé mis en œuvre par ordinateur selon la revendication 1, dans lequel le traitement des données de test sélectionnées comprend :
pour chacune des valeurs de comptage ADC, la génération d'une valeur de concentration de gaz pour les valeurs de comptage ADC.

4. Procédé mis en œuvre par ordinateur selon la revendication 2, dans lequel l'application des données de test sélectionnées comprend :
la lecture de la ou des valeurs de comptage ADC pour le gaz à partir d'un tampon de données de test (124) ; et
la fourniture de la ou des valeurs de comptage ADC à une unité de traitement de données de gaz (112) comprenant le micrologiciel.

5. Procédé mis en œuvre par ordinateur selon la revendication 4, dans lequel le tampon de données de test (124) comprend des valeurs de comptage ADC respectives pour chaque gaz parmi le ou les gaz.

6. Procédé mis en œuvre par ordinateur selon la revendication 1, comprenant en outre la génération d'un rapport de test sur la base, au moins en partie, des données de sortie de test.

7. Procédé mis en œuvre par ordinateur selon la revendication 1, dans lequel le ou les signaux de sortie correspondent à un ou plusieurs parmi : un niveau d'alarme, un niveau de vibration ou un motif d'indicateur lumineux.

8. Procédé mis en œuvre par ordinateur selon la revendication 1, dans lequel l'application des données de test sélectionnées comprend l'application des données de test sélectionnées en réponse à la réception d'un signal de mode de test.

9. Dispositif détecteur de gaz (10) comprenant :
une unité de commande de test (110), dans lequel l'unité de commande de test (110) est configurée pour :
pour chaque gaz parmi un ou plusieurs gaz ;
appliquer les données de test sélectionnées à un micrologiciel du dispositif détecteur de gaz (10), dans lequel les données de test sélectionnées comprennent des données de capteur simulées et sont sélectionnées sur la base, au moins en partie, du gaz ;
générer un ou plusieurs signaux de sortie sur la base, au moins en partie, du traitement des données de test sélectionnées par le micrologiciel ; et
générer des données de test de sortie sur la base, au moins en partie, d'une comparaison du ou des signaux de sortie avec un ou plusieurs signaux de sortie attendus pour les données de test sélectionnées, dans lequel les données de test de sortie comprennent des données indicatives des performances d'une ou plusieurs fonctionnalités du micrologiciel par rapport au gaz.

10. Dispositif détecteur de gaz (10) selon la revendication 9, dans lequel les données de test sélectionnées comprennent une ou plusieurs valeurs de comptage de convertisseur analogique-numérique, ADC, chacune correspondant à une valeur de concentration de gaz.

11. Dispositif détecteur de gaz (10) selon la revendication 9, dans lequel le traitement des données de test sélectionnées comprend :
pour chacune des valeurs de comptage ADC, la génération d'une valeur de concentration de gaz sur la base, au moins en partie, des valeurs de comptage ADC.

12. Dispositif détecteur de gaz (10) selon la revendication 10, dans lequel l'application des données de test sélectionnées pour un gaz particulier comprend :
la lecture de la ou des valeurs de comptage ADC pour le gaz à partir d'un tampon de données de test (124) comprenant les valeurs de comptage ADC pour chaque gaz parmi le ou les gaz ; et
la fourniture de la ou des valeurs de comptage ADC à une unité de traitement de données de gaz (112) comprenant le micrologiciel.

13. Dispositif détecteur de gaz (10) selon la revendication 9, dans lequel l'unité de commande de test (110) est configurée pour générer un rapport de test sur la base, au moins en partie, des données de sortie de test.

14. Dispositif détecteur de gaz (10) selon la revendication 9, dans lequel le ou les signaux de sortie correspondent à un ou plusieurs parmi : un niveau d'alarme, un niveau de vibration ou un motif d'indicateur lumineux.

15. Dispositif détecteur de gaz (10) selon la revendication 9, dans lequel l'unité de commande de test (110) est configurée pour appliquer les données de test sélectionnées en réponse à la réception d'un signal de mode de test.
